# EUROPÄISCHE PATENTSCHRIFT

(11) **EP 3 242 637 B1**
(45) Veröffentlichungstag und Bekanntmachung des Hinweises auf die Patenterteilung: **22.05.2019**
(21) Anmeldenummer: 15767497.9
(22) Anmeldetag: 24.09.2015
(51) Int. Cl.: A61F 2/46, A61B 17/17, A61F 2/38, A61F 2/30

(54) **LEHRE FÜR DIE BESTIMMUNG EINER FÜR EINEN PATIENTEN PASSENDEN IMPLANTATGRÖSSE DES FEMURIMPLANTATS EINER KNIE-ENDOPROTHESE**
JIG FOR DETERMINING A PATIENT-ADAPTED IMPLANT SIZE OF THE FEMORAL IMPLANT OF A KNEE ENDOPROSTHESIS
JAUGE POUR DÉTERMINER LA TAILLE EN FONCTION DU PATIENT DE L'IMPLANT FÉMORAL D'UNE ENDOPROTHÈSE DE GENOU

(30) Priorität: 06.01.2015 DE 102015100049
(43) Veröffentlichungstag der Anmeldung: 15.11.2017
(73) Patentinhaber: Waldemar Link GmbH & Co. KG, 22339 Hamburg (DE)
(72) Erfinder: DMUSCHEWSKY, Klaus, 22397 Hamburg (DE)
(74) Vertreter: Raffay & Fleck
(86) Internationale Anmeldenummer: PCT/EP2015/071982
(87) Internationale Veröffentlichungsnummer: WO 2016/110338

(56) Entgegenhaltungen:
- EP-A1- 2 277 460
- WO-A2-2005/046432
- US-A1- 2005 209 600
- US-A1- 2014 025 081

## Beschreibung

### Technisches Gebiet

Die vorliegende Erfindung betrifft eine Lehre für die Bestimmung einer für einen Patienten passenden Implantatgröße des Femurimplantats einer Knie-Endoprothese mit den Merkmalen des Oberbegriffs des Anspruches 1. Diese Lehre ist dabei insbesondere im Zusammenhang mit der Primärversorgung, also der ersten Versorgung des verschlissenen natürlichen Kniegelenkes mit einer Prothese, einzusetzen.

### Stand der Technik

Es ist hinlänglich bekannt, durch Verschleiß oder sonstige Erscheinungen, wie z.B. Tumorbefall, beschädigte Kniegelenke, die eine normale und insbesondere eine schmerzfreie Bewegung für den betroffenen Patienten nicht mehr ermöglichen, einen Ersatz der Gelenkflächen der beteiligten Knochen, also am distalen Femurende sowie an der dorsalen Tibiaseite, durch entsprechende Implantate zu ersetzen. Hierfür gibt es von verschiedenen namenhaften Anbietern am Markt entsprechende Implantate bzw. Implantatfamilien, die heute routinemäßig betroffenen Patienten eingesetzt werden.

Aufgrund unterschiedlicher anatomischer Verhältnisse bei den jeweils betroffenen Patienten sind verschiedene Implantatgrößen zu verwenden. So haben beispielsweise Patienten unterschiedlich groß ausgebildete Knochenabschnitte bzw. Knochenenden am distalen Femur, aber auch an der dorsalen Tibia. Hierfür halten die Anbieter üblicherweise in den jeweiligen Implantatserien entsprechende Implantate (Femurimplantate zum Ersatz der distalen Femurgelenkflächen, Tibiaimplantate zum Ersatz der dorsalen Tibiagelenkflächen) in unterschiedlichen Größen bereit. Hier sind beispielsweise Sets mit zwischen 5 und 10 unterschiedlichen Implantatgrößen bekannt.

Auch wenn präoperative Diagnostik, insbesondere anhand von Röntgen- oder CT-Aufnahmen, für den behandelnden Arzt und Operateur erste Einschätzungen nicht nur des Befundes des Verschleißes oder der krankhaften Zerstörung des Kniegelenkes, sondern auch der Größenverhältnisse des natürlichen Gelenkapparates geben, so kann dennoch eine exakte Größenbestimmung des zu verwendenden jeweiligen Implantates (Femurimplantat, Tibiaimplantat) allein anhand der präoperativen Diagnostik nicht vorgenommen werden, muss der Operateur unter der Operation hier noch eine entsprechende Auswahl treffen. Zudem verschafft sich der Operateur typischerweise während der Operation erst ein abschließendes diagnostisches Bild und legt dabei neben der zu wählenden Größe des jeweiligen Implantatteils, insbesondere des Femurimplantats, auch die genaue Ausrichtung der Anbringung dieses Implantatteils am Restknochen (insbesondere dem nach den erforderlichen Resektionsschnitten verbleibenden Abschnitt des distalen Femurs) fest. Vielfach wird hier nicht eine entlang der von medial nach lateral verlaufenden Achse ausgerichtete Anordnung des Implantates auf dem durch den distalen Femurschnitt gebildeten Plateau gewählt, sondern eine gegenüber dieser Achse um einen externen Implantatwinkel geneigte Ausrichtung eingestellt, wobei diese Neigung typischerweise in einem Bereich zwischen ca. 0 und 7° liegen kann, typisch geneigt von medial nach lateral.

In einer typischen Vorgehensweise für die Durchführung der vorbereitenden Resektionen für das hinsichtlich der passenden Größe auszuwählende Femurimplantat wird zunächst, nachdem das Kniegelenk des Patienten freigelegt worden ist, in gebeugter Stellung des Knies, also in Flexion (Beugestellung typischerweise etwa 90°), der distale Kondylenschnitt vorgenommen, bei dem an der Stelle, an dem die distalen Kondylen, also Gelenkflächen, des Femurs angeordnet sind, eine erste plan ausgeführte Schnittfläche geschaffen wird. Im Anschluss daran wird typischerweise mit einer wie im Oberbegriff des Anspruches 1 gebildeten Lehre durch Vermessung der anatomischen Dimensionen von anterior nach posterior die Bestimmung der für den Patienten passenden Implantatgröße vorgenommen. Hierzu wird die Lehre mit den Anlagestücken unter die posterioren Kondylen gesetzt, die in der Flexionsstellung auf dem Tibiaplateau liegen, und es wird mit dem Tastarm, genauer mit dessen Tastspitze ein anteriorer Referenzpunkt auf dem Femur angetastet, den der Operateur für passend erachtet, typischerweise der höchstgelegene anteriore, dorsal zu den anterioren Kondylenausläufern gelegene Punkt. Dabei wird das Tasterteil in der Messrichtung entsprechend verschoben, und es lässt sich dann an der Skalierung durch die Lage des Zeigers relativ zu dieser Skalierung eine Implantatgröße ablesen, die für den Patienten geeignet ist.

Beispiele derartiger Lehren sind zusammen mit der entsprechenden Vorgehensweise bei deren Verwendung beschrieben z.B. in der WO 94/00056 A1, der WO 2005/046432 A2, der US 5,624,444, der US 5,810,831, der US 2004/0220583 A1 und der US 2009/0143783 A1.

Weiterhin sind solche Lehren bekannt, bei denen zusätzlich an dem Grundkörper Einrichtungen für die Vorgabe eines externen Rotationswinkels des Implantates vorgesehen sind, wobei diese Einrichtungen Pinlöcher aufweisen, durch die nach Einstellung der gewünschten Rotation entsprechende Pins in die durch den distalen Kondylenschnitt geschaffene erste Anlagefläche des Implantates getrieben werden können, zur weiteren Orientierung und Ausrichtung des Implantates in seiner Winkellage relativ zu einer von medial nach lateral verlaufenden Linie über die entsprechend anhand dieser Pins vorgenommene Orientierung der Schnittlehre für die weiteren für die Formgebung des Femurs vorzunehmenden Resektionsschnitte. Ein Beispiel für eine solche Lehre ist beschrieben in der DE 60 2004 011 420 T2, ein weiteres Beispiel ist die von dem US-amerikanischen Anbieter Zimmer, Inc. in dessen Broschüre "Zimmer® NexGen® CR-Flex and LPS-Flex Knees Surgical Technique with Posterior Referencing Instrumentation" dargestellte Lehre, die an dem Grundkörper neben dem Tasterteil noch einen um einen Schwenkzapfen verschwenkbaren Rotationskörper enthält, mit dem die entsprechende externe Winkeleinstellung vorgegeben werden kann und in dem die oben erläuterten Pinlöcher ausgebildet sind.

Allen oben genannten bekannten Lehren für die Bestimmung der für einen Patienten passenden Implantatgröße des Femurimplantates einer Knie-Endoprothese ist gemein, dass die Anlagestücke für die Anlage an und für die Referenzierung auf jeweils die mediale und die laterale posteriole Kondyle fest mit dem Grundkörper verbunden sind, dass gegenüber diesem für die Größenbestimmung lediglich das Tasterteil in der Messrichtung bewegt werden kann.

Diese Ausgestaltung kann aber, insbesondere wenn zugleich mit der Größenbestimmung die Vorgabe einer externen Winkeleinstellung vorgenommen werden soll, zu Messfehlern und daraus folgend zu Problemen bei der Auswahl der passenden Implantatgröße führen.

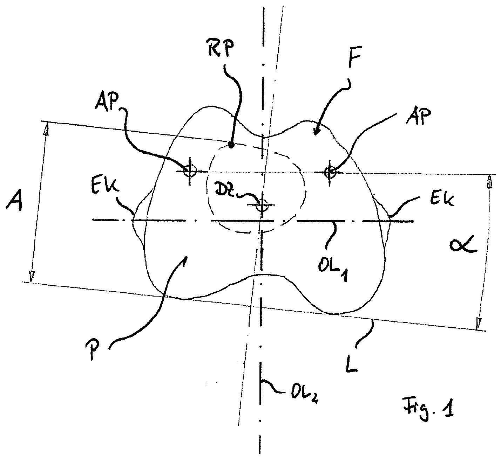

Wie Fig. 1 zeigt, kommt es als Folge der Verkippung gegenüber der feststehenden Messarchitektur zu einer Abweichung der tatsächlichen Abstände in der verkippten Messrichtung und zwar unterschiedlich im Hinblick auf medial oder lateral. In Fig. 1 ist schematisiert eine Aufsicht auf das distale Ende eines Femurs F gezeigt, wobei dieses distale Ende bereits präpariert ist durch einen distalen Schnitt unter Ausbildung einer Planfläche P.

Zunächst einmal sind hier zwei durch die Anatomie vorgegebene, und von den operierenden Ärzten in der Regel als Orientierungshilfe verwendete Orientierungslinien OL1 und OL2 zu erkennen. Die erste dieser Linien, die Orientierungsline OL1 verläuft zwischen den seitlichen Extrempunkten der Epikondylen EK, die zweite Orientierungslinie OL2 verbindet die zwischen der lateralen und der medialen Kondyle gelegenen tiefer gezogenen Flächen in etwa an ihren tiefsten Punkten.

Weiterhin zu erkennen ist ein Referenzpunkt RP, der nach proximal verschoben von dem distalen Ende des Femurs F jenseits der Kondylen am Femurschaft liegt, und an dem ein Operateur mit dem Taster der üblichen Lehre antastet. Die zweite Referenz zur Größenbestimmung bildet die Verbindungslinie der posterioren Kondylen, hier als Linie L eingezeichnet. Hieraus ergibt sich ein senkrecht zu dieser Linie L bestimmter Abstand A zu dem Referenzpunkt RP, der die Größenbestimmung des Femurimplantates vorgibt. Desweiteren ist nun aufgezeigt, dass nach der Größenbestimmung, wie vorstehend beschrieben, eine Festlegung des Implantatwinkels vorgenommen wird, indem eine Rotation um den Winkel, bzw. den Winkelversatz α um ein Drehzentrum DZ vorgenommen wird. Dabei erfolgt die Rotation zur Ausrichtung relativ zu der Linie L, so dass sich auf einer entsprechend um diesen Winkel α gegenüber der Linie L verkippten Linie die Ausrichtpunkte AP ergeben, die später für die Weiterführung der Schnitte entsprechend der um den Implantatwinkel verkippt vorzusehenden Anbindung des Femurimplantates verwendet werden.

Sehr leicht ist nun aber hier zu erkennen, dass die Ausrichtpunkte medial bzw. lateral nun nicht mehr einen einheitlichen Abstand zu den referenzierten posterioren Kondylen aufweisen, dass vielmehr der in der Fig. 1 links dargestellte Ausrichtpunkt AP einen kleineren, der in der Figur rechts dargestellte Ausrichtspunkt AP einen geringeren Abstand zu der jeweiligen posterioren Referenzkondyle aufweist.

Bei dem in Fig. 1 skizzierten und derzeit üblichen Vorgehen wird mit anderen Worten also vor einer Verkippung eine bestimmte Implantatgröße in der Messung von posterior nach anterior ermittelt, die für den Patienten als passend festgelegt wird, und es kommt dann nach der Verkippung zu entsprechenden Fehlern, die nicht erkannt und somit auch nicht mehr korrigiert werden. In der Praxis zeigt sich tatsächlich, dass in diesem Zusammenhang häufig die zuvor bestimmte Implantatgröße nicht zutrifft, so dass entweder noch einmal das Implantat gewechselt und auf eine andere Größe zurückgegriffen werden muss, was mit einer Nachresektion zur Anpassung der entsprechenden Schnitte verbunden ist, oder aber ein erfahrener Operateur hier sogleich anhand seines Erfahrungswertes abweichend von der bestimmten Größe eine nach der Verkippung dann passende Implantatgröße auswählt.

Tatsächlich wird hier also insbesondere aufgrund der lateralen Anordnung des anterioren Referenzpunktes nur auf eine der beiden posterioren Kondylen, z.B. medial, referenziert, anhand dieser Referenz die Größe bestimmt, ohne aber die Gegebenheiten auch auf der zweiten Seite, also in vorstehenden Beispiel im Hinblick auf die laterale posteriore Kondyle, zu berücksichtigen.

Eine Lehre mit den Merkmalen des Oberbegriffs des Anspruchs 1 ist aus der US 2005/0209600 A1 bekannt. Diese Lehre ist jedoch für den anwendenden Arzt schwer zu bedienen, da er eine gleichzeitige Einstellung der beiden Anlagestücke vornehmen muss. Dies ist insbesondere im Umfeld einer Operationsstelle nur schwer möglich. Darstellung der Erfindung

Diesem Fehler zu begegnen und die damit verbundenen Probleme zu lösen, ist Aufgabe der vorliegenden Erfindung.

Gelöst wird diese Aufgabe durch eine Lehre für die Bestimmung einer für einen Patienten passenden Implantatgröße des Femurimplantates einer Knie-Endoprothese mit den Merkmalen des Anspruches 1. Vorteilhafte Weiterbildungen einer erfindungsgemäßen Lehre sind in den abhängigen Ansprüchen 2 bis 9 genannt.

Erfindungsgemäß weist eine Lehre für die Bestimmung einer für einen Patienten passenden Implantatgröße des Femurimplantates einer Knie-Endoprothese zunächst - und dies in Übereinstimmung mit dem Stand der Technik - einen Grundkörper, ein Tasterteil, zwei Anlagestücke sowie wenigstens eine Skalierung und einen zugeordneten Zeiger auf. Dabei weist das Tasterteil einen Tastarm mit einer Tastspitze für die Anlage an einem anterioren Referenzpunkt des distalen Femurendes des Patienten auf und ist das Tasterteil gegenüber dem Grundkörper entlang einer Messrichtung verschiebbar. Insbesondere ist mit Vorteil die Verlagerbarkeit des Tasterteils gegenüber dem Grundkörper - mit Ausnahme einer möglichen Verdreh-, Verschwenk- oder quer zu der Messrichtung möglichen Verschiebbarkeit des Tastarms relativ zu einem weiteren Element des Tasterteils - auf diese lineare Verlagerbarkeit beschränkt, gibt es insbesondere keine Möglichkeit das Tasterteil gegenüber dem Grundkörper in einer Ebene, in der die Messrichtung verläuft, zu schwenken oder zu rotieren.

Die Anlagestücke dienen der Anlage an und der Referenzierung auf jeweils die mediale und die laterale posteriore Kondyle des distalen Femurendes des Patienten. Die Skalierung und der zugeordnete Zeiger dienen schließlich der Anzeige einer Lage des Tasterteils in Messrichtung relativ zu wenigstens einem der Anlagestücke für die Angabe einer passenden Implantatgröße.

Weiterhin sind, auch dies ist bereits aus dem Stand der Technik an sich bekannt, die Anlagestücke relativ zu dem Grundkörper in einer Weise bewegbar, dass ein auf die Messrichtung projizierter Abstand der Tastspitze zu den Anlagestücken für die Anlagestücke derart einstellbar ist, dass dieser Abstand in Bezug auf die beiden Anlagestücke unterschiedlich ist. Dabei sind für jedes Anlagestück eine eigene Skalierung und ein zugeordneter Zeiger für die Anzeige einer Lage des Tasterteils in Messrichtung relativ zu dem jeweiligen Anlagestück und für die sich daraus ergebende Angabe einer passenden Implantatgröße vorgesehen.

In erfindungsgemäßer Weise ist bei der erfindungsgemäßen Lehre zwischen den Anlagestücken eine Kopplung gegeben, die bei einer Relativbewegung des einen Anlagestückes gegenüber dem Grundkörper eine Relativbewegung auch des anderen Anlagestückes gegenüber dem Grundkörper nach sich zieht. Eine solche Kopplung vereinfacht die Handhabung der erfindungsgemäßen Lehre dahingehend, dass der Operateur mit lediglich der Einstellung eines Abstandes eines Anlagestückes gegenüber der Tastspitze auch das zweite Anlagestück nachführt, hier nicht gesondert und von Hand justieren muss. Dabei kann dann eine Grundstellung gegeben sein, in der die beiden Anlagestücke einen gleichen auf die Messrichtung projizierten Abstand zu der Tastspitze aufweisen, wobei durch jede aus der Grundstellung herausführende Relativbewegung zwischen einem ersten der Anlagestücke und dem Grundkörper die Kopplung für eine solche Relativbewegung des zweiten der Anlagestücke und dem Grundkörper sorgt, die zu einer Einstellung der Anlagestücke in der Weise führt, dass diese unterschiedliche auf die Messrichtung projizierte Abstände zu der Tastspitze aufweisen. Auf diese Weise wird eine entsprechende Anpassung an die tatsächlich gegebenen unterschiedlichen Verhältnisse erreicht.

Mit der erfindungsgemäßen Lehre ist der Operateur nun also nicht mehr darauf beschränkt, allgemein einen für beide mit den Anlagestücken abgetasteten Kondylen gleichermaßen gültigen, letztlich - insbesondere bei noch hinzugefügter Rotation - gemittelten auf die Messrichtung projizierten anterioren zu posterioren Abstand der Tastspitze zu den Anlagestücken und somit eine sich daraus ergebende Implantatgröße zu bestimmen. Vielmehr kann der Operateur nun einen entsprechenden Abstand für jedes der Anlagestücke individuell bestimmen, daraus eine entsprechend zu wählende Implantatgröße ablesen. Hierbei kann es nun sein, dass die entsprechende Implantatgröße nach medialer Bestimmung von der entsprechenden Implantatgröße nach lateraler Bestimmung (also der Abstand des an der medialen posterioren Kondyle anliegenden Anlagestückes zu der Tastspitze, projiziert auf die Messrichtung, und der auf die Messrichtung projizierte Abstand des an der lateralen posterioren Kondyle anliegenden Anlagestückes zur Tastspitze) voneinander abweichen. Hier erhält der Operateur jedoch eine wichtige Information, die er in einer von ihm präferierten Weise verwerten kann. So kann er beispielsweise eine aus seiner Sicht zwischen diesen ermittelten Größenwerten liegende, passende Implantatgröße wählen. Er kann andererseits aber auch anhand der vorbestimmten Größe eine externe Implantatrotation wählen, die hier wiederum einen Ausgleich der Größendifferenz herbeiführen kann.

Durch die erfindungsgemäße Lösung ist der Operateur also nicht mehr auf die letztlich lediglich gemittelte und nicht weiter differenzierbare Größenangabe der mit den bekannten Lehren bestimmbaren Implantatgrößen gebunden, sondern kann hier basierend auf einer feiner differenzierten Größenbestimmung (letztlich einer Größenbestimmung für einen medialen Wert und einen lateralen Wert) fundierter eine Auswahlentscheidung für die Implantatgröße und/oder eine Positionierung des Implantates hinsichtlich eines externen Implantatwinkels bestimmen.

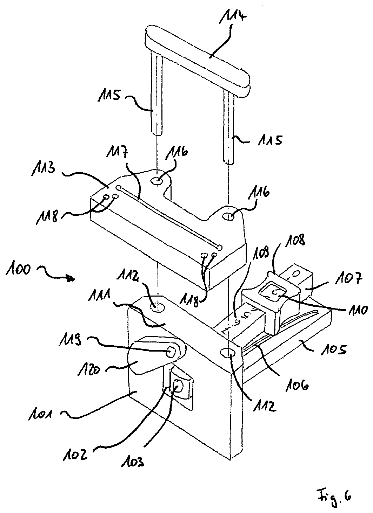

Diese Vorgehensweise kann im Hinblick auf die Operationstechnik noch weiter verfeinert werden, wenn auch bereits bei der Anbringung des distalen Femurschnitts, der bei der Resektion und Vorbereitung des distalen Femurendes für die Aufnahme des Femurimplantates der Knieendoprothese typischerweise zuerst erfolgt, im Hinblick auf die Referenzierung eine der Kondylen (medial oder lateral) ausgewählt wird, die dann in der nachfolgenden Größenbestimmung auch als Referenz beibehalten wird. Dies kann z.B. auf die in der Beschreibung der möglichen Ausgestaltungsformen der Erfindung anhand der Figuren 6 bis 9 erläuterten Weisen geschehen.

Gemäß einer weiteren vorteilhaften Weiterbildung der erfindungsgemäßen Lehre können die Anlagestücke jeweils einen in der Messrichtung verlaufenden, mit dem Anlagestück fest verbundenen Fortsatz aufweisen, wobei die Fortsätze über zwei zueinander parallel verlaufende Querstücke miteinander verbunden sind, wobei die Querstücke in jeweils einem Verbindungspunkt mit dem jeweiligen Fortsatz gelenkig verbunden sind, so dass die beiden Fortsätze und die beiden Querstücke ein Parallelogramm-Vierpunktgelenk bilden. Bei dieser Lösung ist also jeweils ein Querstück an einer erste Verbindungsstelle, z.B. an einem Ende, mit einem ersten der Fortsätze gelenkig verbunden, an einer zweiten Stelle, z.B. einem zweiten Ende, mit dem zweiten der Fortsätze ebenfalls gelenkig verbunden. In einer Weise, dass die beiden Fortsätze parallel zueinander ausgerichtet sind, ist parallel zu dem ersten Querstück das zweite Querstück mit Abstand zu dem ersten Querstück angeordnet und ebenfalls mit einer ersten Verbindungsstelle, insbesondere einem ersten Ende mit dem ersten der Fortsätze gelenkig verbunden, wobei diese gelenkige Verbindung entsprechend einen Abstand zu der gelenkigen Verbindung des anderen Querstückes mit diesem Fortsatz aufweist; ist das zweite Querstück entsprechend mit dem zweiten Fortsatz an einer zweiten Verbindungsstelle, insbesondere an seinem zweiten Ende, mit dem zweiten Fortsatz gelenkig verbunden, wobei auch hier die beiden gelenkigen Verbindungen des ersten und des zweiten Querstückes entsprechend zueinander beabstandet sind. Eine entsprechende Ausbildung der Kopplung zwischen den Anlagestücken über ein durch die Fortsätze und die Querstücke und ihre jeweilige gelenkige Verbindung in den vier Verbindungspunkten gebildetes Parallelogramm-Vierpunktgelenk hat den Vorteil einer einfachen Kopplung, die eine entsprechende Einstellung der Anlagestücke mit unterschiedlichen Abständen zu der Tastspitze ermöglicht. Dies gilt insbesondere dann, wenn die Querstücke, wie gemäß einer weiteren vorteilhaften Ausbildung der Erfindung vorgeschlagen, derart gelenkig mit dem Grundkörper verbunden sind, dass diese Querstücke gekoppelt relativ zu dem Gelenkkörper verschwenkbar sind. Diese gelenkige Verbindung mit dem Grundkörper kann insbesondere so gestaltet sein, dass jedes der Querstücke an einem mittig zwischen den beiden Verbindungspunkten, an denen es gelenkig mit jeweils einem der beiden Fortsätze verbunden ist, gelegenen Anbindungspunkt gelenkig mit dem Grundkörper verbunden ist. Auf diese Weise ergibt sich eine ausgeglichene Auslenkung des jeweiligen gelenkig mit einem der Fortsätze verbundenen Endes der Querstücke nach der einen Richtung (z.B. einer entlang der Messrichtung als oben zu definierenden Richtung), wenn sich die gegenüberliegende Seite des Querstückes, also die Verbindung mit dem jeweils anderen Fortsatz um einen entsprechenden Wert entlang der Messrichtung in einer gegenüberliegenden Richtung (z.B. in einer als entlang der Messrichtung nach unten weisend zu definierenden Richtung) bewegt.

Bei der wie beschriebenen verschwenkbaren Kopplung der Querstücke mit dem Grundkörper kann mit Vorteil wenigstens eine Winkelskala sowie ein mit dieser zusammenwirkender Winkelzeiger zum Anzeigen eines Schwenkwinkels vorgesehen sein, um den die Querstücke relativ zu einer Ausgangsstellung verschwenkt sind. Diese Winkelskala kann einen entsprechenden Wert anzeigen, um den die Querstücke relativ zu einer Ausgangsstellung verschwenkt werden, wobei diese Ausgangsstellung in einer vorteilhaften Ausgestaltung z.B. die in der vorstehend beschriebenen vorteilhaften Weiterbildung genannte Grundstellung sein kann. Die Ausgangsstellung kann dabei insbesondere eine entlang einer lateral zu medial verlaufenden Richtung gelegene Längsorientierung der Querstücke sein.

Hierbei können insbesondere in dem Grundkörper, mit besonderem Vorteil aber in dem Tasterteil entsprechende Pinlöcher vorgesehen sein, durch die dann Pins in die zuvor durch den distalen Kondylenschnitt geschaffene Anlageebene getrieben werden können, um so den durch entsprechendes Verschwenken der Querstücke eingestellten externen Implantatwinkel zu markieren für die nachfolgende Ausrichtung der weiteren Resektionsschnitte durch entsprechende Anordnung der Schnittführungsschablonen in dem voreingestellten Winkel durch Aufsetzen mit entsprechend in der Schnittführungsschablone ausgebildeten Pinlöchern über die zuvor wie vorstehend beschrieben in die erste Schnittebene getriebenen Pins.

Da sich aufgrund des Parallelogramm-Vierpunktgelenkes mittels dessen die Anlagestücke in ihrer möglichen Relativbewegung gegenüber dem Grundkörper und damit auch gegenüber der Tastspitze gekoppelt sind, sowohl eine entsprechende Verstellung des auf die Messrichtung projizierten Abstandes zwischen der Tastspitze und dem jeweiligen Anlagestück (medial bzw. lateral) ergibt als auch eine Einstellung des externen Implantatwinkels, kann über diese Maßnahme der Operateur den Zusammenhang zwischen den unterschiedlichen angezeigten Implantatgrößen und dem vorgegebenen externen Implantatwinkel bestimmen und z.B. den externen Implantatwinkel so wählen, dass die für medial und lateral angezeigten Implantatgrößen bestmöglich in Übereinstimmung stehen, um so optimal eine Größenwahl des Femurimplantates vornehmen zu können. Alternativ kann aber der Operateur auch einen von ihm bevorzugten externen Implantatwinkel vorgeben und dann die mediale und laterale Implantatgröße aus der jeweiligen Skala ablesen und bei unterschiedlichen Ergebnissen anhand seiner eigenen Erfahrung eine für seine Operationstechnik passende Implantatgröße auswählen.

Um dem Operateur das Handhaben der erfindungsgemäßen Lehre zu vereinfachen, kann mit Vorteil ein Handgriff vorgesehen sein, der insbesondere mit dem Grundkörper verbunden sein kann.

Desweiteren kann ein wie gemäß einer weiteren vorteilhaften Weiterbildung der Erfindung vorgeschlagenes Blockiermittel zum Blockieren von zueinander bewegbaren Teilen der Lehre den Operateur darin unterstützen, die einmal angewählte Position der Lehre hinsichtlich der eingestellten, auf die Messrichtung projizierten Abstände der beiden Anlagestücke und - sofern vorhanden - hinsichtlich einer Verkippung der Ausrichtungslage lateral zu medial festzustellen, um einen einmal eingestellten Wert festzuhalten, diesen ggf. auch nach Entfernen der Lehre aus der Anlage an der Schnittebene des distalen Kondylenschnittes noch einmal nachvollziehen zu können. Ein solches Blockiermittel kann z.B. eine Feststellschraube sein.

Weiterhin kann mit Vorteil die erfindungsgemäße Lehre zerlegbar sein, um diese nach Gebrauch einfach reinigen und desinfizieren zu können. Die Zerlegbarkeit ist dabei insbesondere mit Vorteil ohne Einsatz von Werkzeug gegeben, indem Einzelteile beispielsweise durch kraftschlüssige Schnappverbindungen oder durch formschlüssige, in einer bestimmten Stellung voneinander lösbare Verbindungen, miteinander verbunden sind.

Einzelne Bewegungsbereiche der relativ zueinander bewegbaren Teile der Lehre können insbesondere durch zwischen diesen Teilen gebildete Anschläge begrenzt sein. Auf diese Weise können typischerweise als sinnvolle Bereiche angesehene Bewegungsbereiche bestimmt werden, die nicht überschritten werden können. Dies unterstützt den Operateur bei dem Einsatz der erfindungsgemäßen Lehre, da er nicht etwa darauf achten muss, mit dieser Lehre die sinnvollen Bereiche einer Einstellung nicht zu überschreiten.

Weitere Vorteile und Merkmale der Erfindung ergeben sich aus der nachfolgenden Beschreibung anhand eines Ausführungsbeispiels unter Bezugnahme auf die entsprechenden, dieser Anmeldung beigefügten Figuren. In den Figuren zeigen:

### Kurze Beschreibung der Zeichnungen

Fig. 1 eine schematische Darstellung zur Verdeutlichung des Problems des Messfehlers, wie er bei bekannten Lehren nach dem Stand der Technik vorzufinden ist;
Fig. 2 in einer Explosionsdarstellung ein Ausführungsbeispiel einer erfindungsgemäßen Lehre mit individuell in ihrem auf die Messrichtung projizierten Abstand zu der Tasterspitze einstellbaren Anlagestücken;
Fig. 3 eine Seitenansicht einer nach Anbringen des distalen Resektionsschnittes auf das distale Femurende aufgesetzten erfindungsgemäßen Lehre nach Fig. 2;
Fig. 4 eine Frontalansicht auf das distale Ende des Femurs mit in Flexion befindlicher Stellung des Knies und auf das distale Ende auf den dort bereits angebrachten Resektionsschnitt, also auf die Schnittebene, aufgesetzter Lehre gemäß dem Ausführungsbeispiel nach Fig. 2;
Fig. 5 eine vereinfachte schematische Darstellung lediglich der Kopplung der beiden Anlagestücke durch das Parallelogramm-Vierpunktgelenk und die damit erreichte unterschiedliche Abstandseinstellung zu dem mit der Tastspitze des Tasters angetasteten Referenzpunkt, also zu der Lage der Tastspitze;
Fig. 6 ein für eine vorteilhafte gezielte Referenzierung auf eine ausgewählte der Kondylen (medial oder lateral) verwendbares Ausrichtinstrument für den distalen Femurschnitt zur Verwendung im Operationsinstrumentarium zusammen mit einer erfindungsgemäßen Lehre in einer dreidimensionalen, z.T. in explodierter Form gezeigten Darstellung;
Fig. 7 in zwei Darstellungen a) und b) das Ausrichtinstrument der Fig. 6 in Anwendung zur Ausrichtung gezielt auf die mediale bzw. die laterale Kondyle;
Fig. 8 in einer den Darstellungen in Fig. 7 vergleichbaren Darstellung ein alternatives Ausrichtinstrument für den distalen Femurschnitt in Anwendung und
Fig. 9 eine Messlehre für eine Größenbestimmung des Abschnitts einer Kondyle für die Unterstützung bei der Implantatgrößenbestimmung.

### Weg(e) zur Ausführung der Erfindung

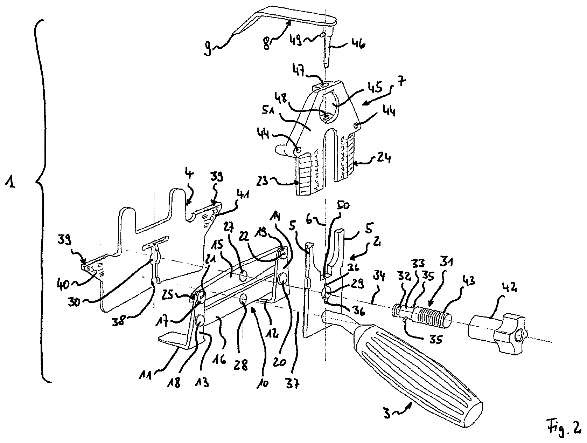

In Fig. 2 ist skizzenhaft in einer Explosionsdarstellung ein Ausführungsbeispiel einer erfindungsgemäßen Lehre für die Bestimmung einer für einen Patienten passenden Implantatgröße des Femurimplantates einer Knie-Endoprothese gezeigt. Diese Lehre, die allgemein mit dem Bezugszeichen 1 bezeichnet ist, dient in dem gezeigten Ausführungsbeispiel nicht nur der Größenbestimmung des Implantates, sondern auch der Bestimmung bzw. der Vorgabe der Ausrichtung nach dem externen Implantatwinkel.

Die Lehre 1 weist zunächst ein Halte- und Führungsteil 2 auf, an dem ein Handgriff 3 fest angeformt ist. Mit dem Halte- und Führungsteil 2 im Gebrauch fest und relativ zueinander unverrückbar verbunden ist ein Gegenstück 4. Das Halte- und Führungsteil 2 und das Gegenstück 4 bilden insoweit einen Grundkörper der Lehre 1.

Auf an dem Halte- und Führungsteil 2 ausgebildeten, seitlich vorragenden Führungsschienen 5 in einer Messrichtung entlang der Linie 6 verschiebbar aufgesetzt ist ein Tasterteil 7. Bestandteil des Tasterteils 7 und an einem Schlittenelement 51 desselben lösbar angeordnet und relativ zu diesem um die Achse 6 verschwenkbar ist ein Tastarm 8, der an einem abgeknickt verlaufenden Ende an dessen vorderster Kante eine Tastspitze 9 aufweist. Dabei ist diese Tastspitze 9 in dem gezeigten Ausführungsbeispiel nicht etwa punktförmig zugespitzt, sondern bildet vielmehr einen kanten- bzw. linienförmigen Verlauf, der jedoch hier ebenfalls als "Tastspitze" bezeichnet wird, da mit ihm ein anteriorer Referenzpunkt angetastet wird.

Weiterhin zu erkennen ist ein Anlageteil 10. Dieses enthält zwei Anlagestücke 11, 12, die hier in etwa plattenförmig ausgebildet sind. Die Anlagestücke 11, 12 sind einstückig mit in etwa rechtwinklig zu diesen verlaufenden Fortsätzen 13, 14 verbunden. Dabei sind die Fortsätze 13, 14 parallel zueinander orientiert und verlaufen im zusammengesetzten Zustand der Lehre 1 parallel zu der durch die Achse 6 vorgegebenen Messrichtung.

Zwischen den Fortsätzen 13, 14 sind zwei wiederum in ihrem Längsverlauf parallel zueinander angeordnete Querstücke 15, 16 angeordnet, die jeweils in Verbindungspunkten 17, 18, 19, 20 gelenkig mit den Fortsätzen 13, 14 verbunden sind. In dieser Ausgestaltung bilden die Fortsätze 13, 14 mit den Querstücken 15, 16 ein Parallelogramm-Vierpunktgelenk.

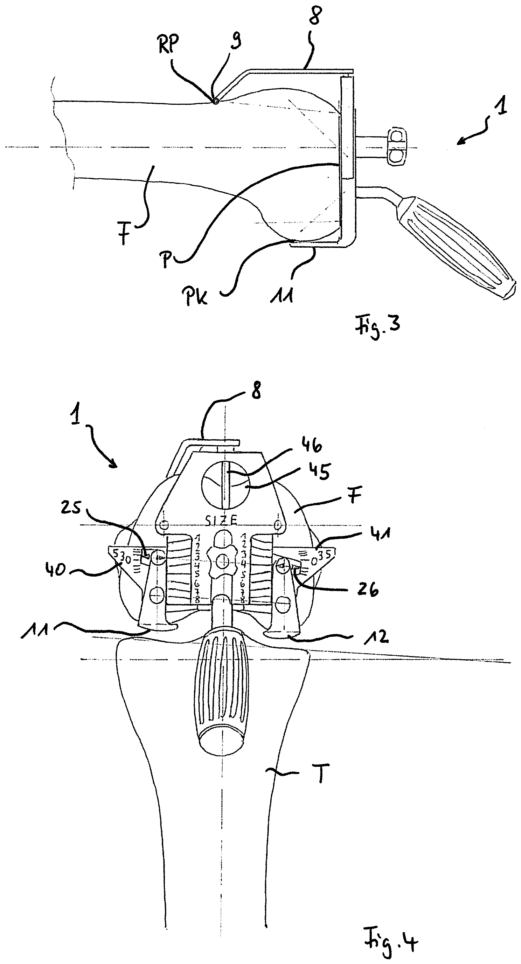

Im Bereich der Verbindungspunkte 17, 19 sind Zeigermarkierungen 21, 22 angebracht, die im zusammengefügten Zustand der Lehre 1 jeweils einer linken und einer rechten Skalierung 23, 24 auf dem Tasterteil 7 gegenüberliegen und einer entsprechenden Anzeige auf der jeweiligen Skalierung 23 bzw. 24 dienen. Eine weitere Zeigermarkierung 25 ist auf einem seitlichen Überstand des Querstückes 15 im Bereich des Fortsatzes 13 zu erkennen, wobei auf der gegenüberliegenden Seite an einem weiteren seitlichen Überstand des Querstückes 15 eine weitere solche Zeigermarkierung 26 angebracht ist, die in Fig. 4 erkannt werden kann.

Schließlich ist hier zu sehen, dass in den Querstücken 15, 16 jeweils mittig zwischen den einander gegenüberliegenden Verbindungspunkten 17, 19 bzw. 18, 20 Durchtrittsöffnungen 27 bzw. 28 eingebracht sind. Die Durchtrittsöffnung 27 liegt im zusammengesetzten Zustand der Lehre 1 in Flucht mit einer weiteren Durchtrittsöffnung 29 in dem Halte- und Führungsteil 2 und einer Klemmöffnung 30 in dem Gegenstück 4. Zum Zusammenbau ist ein Halte- und Schraubbolzen 31 durch die Durchtrittsöffnung 29, die Durchtrittsöffnung 27 und in die Klemmöffnung 30 hineingeführt. Dort ist dieser Halte- und Schraubbolzen mit einem an seinem ersten Ende gelegenen Hinterschnitt 32 festgelegt. Auf einem glattwandigen Abschnitt 33 des Halte- und Schraubbolzens ruht die Durchtrittsöffnung 27, so dass diese um die durch den Halte- und Schraubbolzen 31 definierte und mit 34 gezeigte Achse rotieren kann. Seitliche Verriegelungsstifte 35 passen in einer ersten Drehstellung des Halte- und Schraubbolzens 31 durch entsprechende Ausnehmungen 36 am Rande der Durchtrittsöffnung 29 und verriegeln, wenn der Halte- und Schraubbolzen 31 aus der Flucht der Verriegelungsstifte 35 mit den Ausnehmungen 36 verdreht wird, das Halte- und Führungsteil 2. Auf der dem Anschluss des Handgriffes 3 an dem Halte- und Führungsteil 2 gegenüberliegenden Seite ist dort fest angeformt ein weiterer, in dieser Darstellung nicht erkennbarer Klemmzapfen, der sich entlang einer weiteren Achse 37 erstreckt und im zusammengesetzten Zustand durch die Durchtrittsöffnung 28 in dem Querstück 16 ragt und mit einem dem Hinterschnitt 32 an dem Halte- und Schraubbolzen 31 vergleichbaren Hinterschnitt in einer weiteren Klemmöffnung 38 in dem Gegenstück 4 verklemmt sitzt.

Das Gegenstück 4 weist an zwei zeigerartig geformten, nach außen weisenden Seitenwangen 39 jeweils eine Winkelskalierung 40, 41 auf, die mit den Zeigermarkierungen 25 bzw. 26 zusammenwirken, wie dies insbesondere in Fig. 4 zu erkennen ist, um die Einstellung eines externen Implantatwinkels anzuzeigen. Die zeigerartig zugespitzte Form der Seitenwangen 39 soll dem Operateur bei einer intuitiven Ausrichtung des mit dem Halte- und Führungsteil 2 verbundenen Gegenstückes 4 dienen, insbesondere zum Fluchten einer gedachten Verbindungslinie zwischen der medialen und der lateralen Epikondyle.

Eine Schraubmutter 42 mit einem Innengewinde kann auf ein auf dem Halte- und Schraubbolzen 31 ausgebildetes Außengewinde 43 aufgeschraubt werden, um insbesondere bei einem festen Anziehen der Schraubmutter 42 einen Druck auf die dem Handgriff 3 zugewandte Vorderseite des Halte- und Führungsteils 2 bzw. einen Zug auf das Gegenstück 4 auszuüben und so die relativ zueinander in der nachfolgend noch näher geschilderten Weise bewegbaren Teile in einer eingenommenen Relativposition zueinander zu verklemmen (dies gilt nicht für den relativ zu dem Schlittenelement 51 verschwenkbaren Tastarm 8 des Tasterteils 7). Schließlich sind an dem Tasterteil 7 zwei Pinlöcher 44 und eine Peilöffnung 45 zu erkennen. Die Pinlöcher 44 dienen dem positionsgenauen Setzen von Markierungspins nach erfolgter Einstellung der Lehre 1, die Peilöffnung 45 hilft dem Operateur, durch diese hindurch einen Schwenkstift 46 des Tastarmes 8, der zentral durch die Peilöffnung 45 verläuft, anzupeilen und so die erfindungsgemäße Lehre auszurichten bzw. deren Ausrichtung zu überprüfen, indem er auf die anteriore interkondylare Fossa zielt.

Zum Zusammensetzen der erfindungsgemäßen Lehre 1 wird das Schlittenelement 51 des Tasterteils 7 auf die Führungsschienen an dem Halte- und Führungsteil 2 aufgesetzt, es wird der Halte- und Schraubbolzen 31 durch die Durchtrittsöffnung 29 und die Durchtrittsöffnung 27 hindurchgeführt, und es wird auch der in der Fig. 2 nicht näher erkennbare, auf der gegenüberliegenden Seite des Anschlusses des Handgriffes 3 an dem Halte- und Führungsteil 2 angeformte Zapfen durch die Durchtrittsöffnung 28 geführt. Sodann wird das Gegenstück 4 auf den Enden des Halte- und Schraubbolzens 31 und des hier nicht gezeigten Verbindungszapfens 38 aufgeklemmt, und es wird schließlich die Schraubmutter 42 auf das Außengewinde 43 des Halte- und Schraubbolzens 31 aufgeschraubt. Der Tastarm 8 wird mit seinem Schwenkstift 46 in dafür vorgesehene Schwenklageröffnungen 47, 48 in dem Schlittenelement 51 eingesetzt und durch Verdrehen eines Verriegelungsstiftes 49 aus einer Einbauposition heraus in eine Gebrauchsposition gesichert. Dabei verhindert nun das untere Ende des Schwenkstiftes 46, dass auf einer Anschlagfläche 50 anschlägt, dass das Tasterteil 7 entlang der Messrichtung (Achse 6) in der Fig. 2 in einer Richtung nach unten weiter verlagert werden kann bis zu einer Ausbauposition. Entsprechend ist die Lehre 1 in dieser zusammengefügten Position gesichert.

Während des Gebrauches kann nun nicht nur das Tasterteil 7 in einer entlang der Achse 6, der Messrichtung, verlagerbaren Position relativ zu dem aus dem Halte- und Führungsteil 2 und dem Gegenstück 4 gebildeten Grundkörper verschoben werden, es können weiterhin relativ zu diesem Grundkörper die Anlagestücke 11, 12 bewegt werden und dies in einer Weise, dass sich für die Anlagestücke 11, 12 unterschiedliche Höheneinstellungen bzw. auf die Messrichtung (Achse 6) projizierte Abstandswerte zu der Tastspitze 9 ergeben. Diese Einstellung erfolgt durch ein Bewegen des Vierpunkt-Parallelogrammgelenkes, welches durch ein Verschwenken der Querstücke 15 bzw. 16 um die durch die Durchführungsöffnungen 27 bzw. 28 geführten Halte- und Verbindungsbolzen 31 und (nicht gezeigt) Verbindungszapfen gebildeten Drehlager um die Achsen 34 bzw. 37 verschwenken. Diese Schwenkbewegung ergibt dabei zugleich eine aus einer medial zu lateral entlang der Epikondylen fluchtend ausgerichteten Normalposition um einen Winkel verlagerte Einstellung des externen Implantatwinkels. Im Gebrauch kann dieser Schwenkwinkel dann im Zusammenwirken der Zeigermarkierungen 25, 26 mit den Winkelskalierungen 40, 41 und kann die jeweilige Implantatgröße in Bezug auf das Anlagestück 11 bzw. das Anlagestück 12 auf der zugeordneten Skalierung 23 bzw. 24 durch die Anzeige der Zeigermarkierungen 21 bzw. 22 abgelesen werden.

In Fig. 3 ist nun schematisch und in einer Seitenansicht die erfindungsgemäße Lehre 1 dargestellt, wie sie gegen eine Planfläche P, die durch den bereits ausgeführten distalen Kondylenschnitt erzeugt ist, gesetzt ist und mit dem Tastarm 8 und der daran angeordneten Tastspitze 9 einen Referenzpunkt RP anterior auf dem Femur F antastet. Zu erkennen ist hier auch, wie das Anlagestück 11 an einer posterioren Kondyle PK anliegt und die Lehre 1 auf diese referenziert.

In Fig. 4 ist die Situation aus Fig. 3 noch einmal in einer Aufsicht auf das distale Ende des Femurs F gezeigt, wobei hier zur Verdeutlichung, dass die Anbindung der erfindungsgemäße Lehre 1 in Flexion erfolgt, auch der dorsale Abschnitt der Tibia T mit dargestellt ist. Zu erkennen ist hier auch, wie durch die Peilöffnung 45 der Schwenkstift 46 des Tastarmes 8 auf die Fossa der anterioren Kondylenfortsätze zeigt und somit eine Ausrichthilfe für den Operateur gibt.

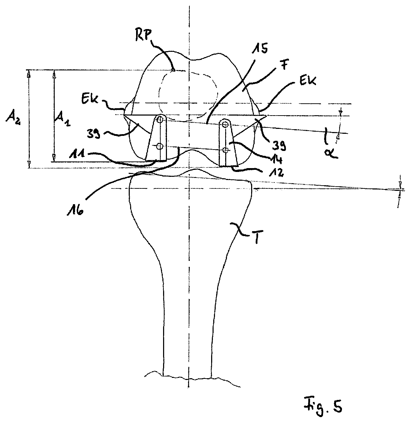

In der Fig. 5 ist schematisch eine Aufsicht auf das distale Ende des Femurs gezeigt mit dazu dargestelltem proximalen Ende der Tibia, wobei auf der bereits auf den distalen Kondylenschnitt präparierten Anlagefläche die dort anliegende Lehre 1 nur durch und reduziert auf die für die Erläuterung anhand dieser Figur und der darin aufgezeigten Zusammenhänge wesentlichen Elemente wiedergegeben ist. Gezeigt sind lediglich die Seitenwangen 39 des Gegenstückes 4, welches Teil des Grundkörpers ist sowie - dies stark schematisiert - das Parallelogramm-Vierpunktgelenk bestehend aus den Fortsätzen 13 und 14 sowie den hier lediglich schematisch als Linien dargestellten Querstücken 15, 16. Weiterhin angedeutet sind die Anlagestücke 11 und 12, die an den posterioren Kondylen medial und lateral angeordnet sind.

In dieser Darstellung kann zum einen erkannt werden, wie die zeigerförmig spitz zulaufenden Seitenwangen 39 für den Operateur zu einer weiteren Orientierung der Positionierung der Lehre 1 dienen, indem diese nämlich mit der Verbindungslinie der beiden Epikondylen EK, der medialen Epikondyle und der lateralen Epikondyle, ausgerichtet sind. Weiterhin ist zu erkennen, dass durch eine entsprechende relative Verlagerung des Parallelogramm-Vierpunktgelenkes gegenüber dem Grundkörper, zu dem die Seitenwangen 39 einen Bestandteil bilden, ein Winkelversatz α gebildet ist, der sich auch in einer unterschiedlichen Höhe bzw. einem unterschiedlichen Abstand der Ansatzstücke 11 und 12 zu dem Referenzpunkt RP, an dem die Tastspitze 9 antastet (vgl. Fig. 3) ausdrückt. Das Ansatzstück 11, welches beispielsweise in dem hier dargestellten Beispiel lateral angeordnet sein kann, weist zu dem Referenzpunkt entlang der Messlinie einen Abstand A₁ auf, der erkennbar geringer ist als der Abstand A₂, den das in diesem Beispiel z.B. lateral angeordnete Ansatzstück 12 zu dem Referenzpunkt RP projiziert auf die Messrichtung aufweist. Durch die besondere Ausgestaltung des Parallelogramm-Vierpunktgelenkes, zu dem die Anlagestücke 11, 12 über die Fortsätze 13, 14 und die Querstücke 15, 16 verbunden sind und welches in der wie vorstehend beschriebenen Weise verdrehbar an dem aus Halte- und Führungsteil 2 und Gegenstück 4 gebildeten Grundkörper angelenkt ist, wird hier also eine nach der jeweiligen Anlagefläche (medial bzw. lateral) unterschiedene Abstandshöhe (der Abstand zum Referenzpunkt RP projiziert auf die Messrichtung) bestimmt und somit für beide der Seiten ein passender Wert für die Größenbestimmung des Implantates. Durch entsprechendes Nachführen der Winkeleinstellung kann der Operateur hier die Parameter so wählen, dass die Größenvorgaben nah beieinander liegen, so dass er eine optimale und passende Implantatgröße und ein entsprechendes Femurimplantat auswählen und für dieses dann sogleich die geeignete Lage bezogen auf den externen Implantatwinkel bestimmen und festlegen kann.

Um die Vorgehensweise, die durch die Verwendung der erfindungsgemäßen Lehre ermöglicht wird, nämlich die gezielte Referenzierung bei der Größenbestimmung des Femurimplantates auf eine der beiden Kondylen (lateral oder medial) insgesamt zu unterstützen bzw. die gesamte Operationstechnik weiter an dieses grundsätzliche Konzept der konkreten Referenzierung auf entweder die laterale oder die mediale Kondyle abzustimmen, können bereits beim Setzen des distalen Kondylenschnittes bzw. im Zusammenhang mit dem Anbringen dieses Schnittes entsprechende Maßnahmen einer ausgewählten Referenzierung unternommen werden.

Eine erste Möglichkeit, dies zu tun, besteht darin, ein wie in Fig. 6 dargestelltes, modifiziertes Ausrichtinstrument zum Ausrichten einer Schneidführung für den distalen Kondylenschnitt zu verwenden.

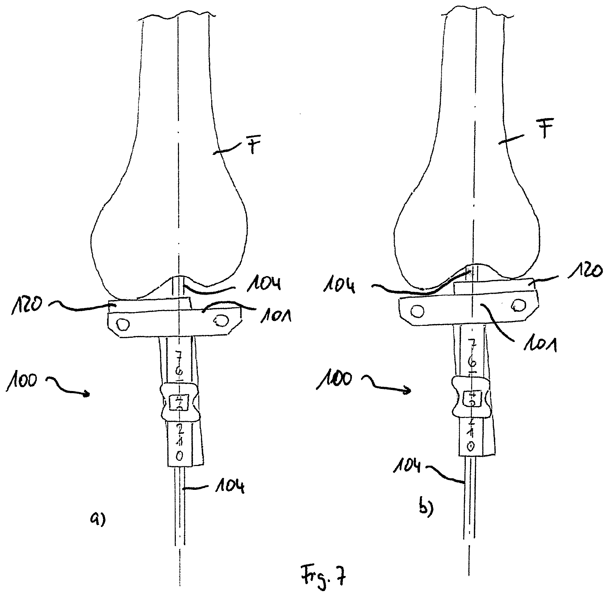

Das in Fig. 6 gezeigte Ausrichtinstrument ist allgemein mit dem Bezugszeichen 100 versehen und dort in einer dreidimensionalen Ansicht gezeigt. Es weist eine Anlageplatte 101 zum in Kontaktbringen mit den distalen Kondylen eines Femurs auf. Die Anlageplatte 101 hat eine zentrale Öffnung 102 durch die hindurch eine als langgestreckte Öffnung gebildete Stangenaufnahme 103 zugänglich ist. Diese Stangenaufnahme 103 dient der Aufnahme einer typischerweise beim Orientieren und Ausrichten des Ausrichtinstrumentes 100 selbst verwendeten, in eine axiale Bohrung in den distalen Femur eingebrachte Intramedullärstange 104 (vgl. Fig. 7). Das in Fig. 6 gezeigte Ausrichtinstrument 100 verfügt dabei noch über eine Winkelverstellmöglichkeit zum Einstellen eines Winkelversatzes relativ zu einer senkrechten Richtung zur Achsrichtung der Intramedullärstange 104 für die einzustellende Schnittebene. Diese Winkelverstellung ist gelöst über einen an der Anlageplatte 101 festgelegten Fortsatz 105, der gekrümmte Führungsnuten 106 auf seiner in der Darstellung in Fig. 6 nach oben weisenden Oberfläche aufweist (hier nur eine der Führungsnuten 106 zu erkennen). Das Element, in der die Stangenaufnahme 103 eingebracht ist, eine Führungsleiste 107, trägt einen auf dieser Führungsleiste 107 längs verschieblichen Schieber 108. Auf seiner der Oberseite mit den Führungsnuten 106 des Fortsatzes 105 zugewandten Seite weist der Schieber einen zapfenartigen Vorsprung auf, mit dem dieser in einer der Führungsnuten 106 eingreift und somit durch die Führungsnut 106 geführt ist. Durch eine Längsverschiebung des Schiebers 108 entlang der Führungsleiste 107 wird somit eine geführte relative Verschwenkung zwischen dem Fortsatz 105 und der Führungsleiste 107 erreicht, so dass die Führungsleiste 107 in einer hier nicht näher dargestellten gelenkigen Lagerung in der Öffnung 102 in einer Winkeleinstellung relativ zu der Anlageplatte 101 verschwenken kann. Auf der in der Fig. 6 oben dargestellten Oberseite weist die Führungsleiste 107 eine Winkelskala 109 auf. Diese kann durch eine Ableseöffnung 110 in dem Schieber 108 abgelesen, und es kann so der eingestellte Schwenkwinkel bestimmt werden.

Quer zu einer Oberkante 111 der Anlageplatte 101 sind zwei Befestigungsöffnungen 112 eingebracht. Diese dienen zum Festlegen eines Schnittführungsblockes 113 am dem Ausrichtinstrument 100. Mit einem Doppelstiftriegel 114, der zwei parallel verlaufende Befestigungsstifte 115 aufweist, wird der Schnittführungsblock 113 auf der Oberkante 111 fixiert, indem die Befestigungsstifte 115 des Doppelstiftriegels 114 durch korrespondierende Öffnungen 116 in dem Schnittführungsblock 113 hindurch bis in die Befestigungsöffnungen 112 hineingeführt werden. Auf diese Weise ist dann der Schnittführungsblock 113 auf der Oberkante 111 festgelegt und damit auch ein als Schnittführung für die Sägeklinge eines Schneidinstrumentes vorgesehener Führungsschlitz 117 in einer entsprechenden Ausrichtung und Orientierung zum distalen Femur bestimmt. Indem durch an dem Schnittführungsblock 113 vorgesehene Pinlöcher 118 Befestigungspins hindurchgeführt und in das Knochenmaterial des distalen Femurendes getrieben werden, kann der Schnittführungsblock 113 auch direkt am Knochen fixiert werden, so dass in üblicher Weise das Ausrichtinstrument 100 und auch der Doppelstiftriegel 114 entfernt werden können, bevor der distale Resektionsschnitt unter Führung durch den Führungsschlitz 117 vorgenommen wird.

In den bis hierhin beschriebenen Elementen und Funktionen stimmt das in Fig. 6 gezeigte und vorstehend beschriebene Ausrichtinstrument 100 mit herkömmlichen und bekannten Ausrichtungsinstrumenten überein. Neu und für die wie oben beschriebene Anpassung an eine neuartige Operationstechnik mit bewusster Referenzierung auf eine ausgesuchte der Kondylen vorgesehen ist ein an der Anlageplatte an einem Gelenkpunkt 119 verschwenkbar festgelegtes, eine deutliche Stärke aufweisendes und insoweit nahezu blockartig gebildetes Anlagestück 120.

Dieses Anlagestück 120, das allgemeiner als Anlage- bzw. Referenzierungselement bezeichnet werden kann und dabei ganz unterschiedlich und von der in der Fig. 6 gezeigten Ausgestaltungsform abweichende Formen annehmen kann, kann zwischen der wie in Fig. 6 gezeigten Position und wenigstens einer weiteren Position, in der das Anlagestück 120 verlagert ist hin zu einer anderen Seite der Anlageplatte 101 verschwenkt werden, um so unter ausgewählter Referenzierung einer der beiden Kondylen, nämlich der medialen Kondyle oder der lateralen Kondyle, gezielt gegen diese ausgewählte Kondyle in Anlage gebracht zu werden.

Dargestellt ist dies in den Figuren 7a und b, wo das Ausrichtinstrument 100 in Verwendung gezeigt ist in zwei Beispielen an einem Femur F des linken Beines. Dabei ist in der Fig. 7a durch entsprechendes Orientieren der Schwenkposition des Anlagestückes 120 dieses so eingestellt, dass es an der lateralen Kondyle anliegt, somit bei der Ausrichtung auf diese laterale Kondyle referenziert wird. In der Darstellung in Fig. 7b ist das Anlagestück 120 in seine zweite Position verschwenkt worden, in der es nun an der medialen Kondyle anliegt, entsprechend die Ausrichtung unter Referenzierung auf diese vorgenommen wird. Wie gut zu erkennen ist, liegt das Ausrichtinstrument 100 mit seiner Anlageplatte 101 in der jeweiligen Ausrichtung des Anlagestückes 120 nicht an der von dem Ausrichtstück 120 nicht kontaktierten Kondyle an, so dass an dieser Stelle keine Referenzierung stattfindet, die Referenzierung sich also ausschließlich über die durch entsprechende Ausrichtung des Anlagestückes 120 referenzierte Kondyle ergibt. Klar zu erkennen ist weiterhin, dass sich aufgrund der unterschiedlichen Referenzierung in Figuren 7a und 7b unterschiedliche Abstände des Ausrichtinstrumentes 100 zu dem Femur F ergeben, was eine unterschiedliche Lage der Schnittebene des distalen Femurschnittes, die mit dieser Ausrichtung eingestellt wird, in Richtung distal zu dorsal einhergeht.

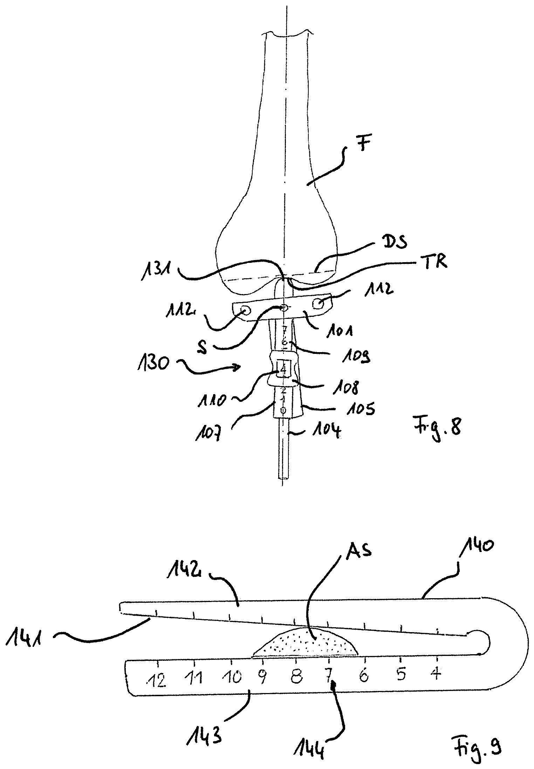

Ein weiteres Mittel bzw. eine weitere Möglichkeit, hier bereits bei der Planung des distalen Resektionsschnittes eine Referenzierung auf eine der Kondylen mit einzubeziehen ist anhand der weiteren Figuren 8 und 9 dargestellt. In Fig. 8 ist dabei ein alternatives Ausrichtinstrument 130 dargestellt, wie es verwendet wird, um am distalen Femur F die Schnittlinie für den distalen Resektionsschnitt DS auszurichten und zu planen. In dem Grundaufbau gleicht dieses Ausrichtinstrument 130 dem zuvor anhand der Figuren 6 und 7 beschriebenen Ausrichtinstrument 100. Entsprechend im Aufbau gleiche Teile sind daher mit gleichen Bezugszeichen auch in Fig. 8 bei dem Ausrichtinstrument 131 eingezeichnet. Auch hier ist auf eine Intramedullärstange 104 eine Führungsleiste 107 aufgeschoben. Relativ zu dieser Führungsleiste 107 um eine Schwenkachse S (in der Figur senkrecht zur Zeichenebene verlaufend) angeordnet ist ein aus einem Ansatz 101 mit einem Fortsatz 105 gebildeter Korpus, der durch Verschwenken um die Schwenkachse S in seiner Winkeleinstellung relativ zu der Ausrichtung der Intramedullärstange 104 justiert werden kann. Hierzu dient, in wie oben anhand des Beispiels nach Fig. 6/7 beschriebener Weise, der Schieber 108, der über die Führungsleiste 107 linear bewegt werden kann und der eine Winkelskala 109 aufweist, anhand derer durch die Ableseöffnung 110 in dem Schieber 109 der eingestellte Winkel einfach abgelesen werden kann. Das Besondere an diesem Ausrichtinstrument 130 ist nun die Art der Referenzierung. Diese Erfolgt über eine in Verlängerung der Anlageplatte 101 (die dann also nicht mehr tatsächlich an den Kondylen zur Anlage kommt) hinaus entlang der Achse, entlang derer die Intramedullärstange 104 verläuft, geführte Abtastspitze 131. Diese ist so gebildet, dass sie mit ihrem vorderen, der Anlageplatte 101 abgewandten Ende die Trochlea TR des distalen Endes des Femur F antastet. Bei dieser Art der Referenzierung auf die Trochlea TR wird darauf abgestellt, dass dieser Bereich (der Bereich der Trochlea TR) bei der Resektion nicht unbearbeitet bleiben soll. Es soll nämlich der dortige Bereich der Patellagleitrinne nicht unbearbeitet bleiben, um insbesondere eine Distalisierung der Implantatlage bzw. der Patellagleitrinne zu vermeiden. Bei dieser Vorgabe wird also de facto die Patellagleitrinne, wie in der Fig. 8 gezeigt, als distale Referenz verwendet.

Wird nach einer derartigen Referenzierung und Festlegung der Resektionsebene die Resektion nun mithilfe eines an der Anlageplatte 101 wie bei dem Ausrichtinstrument 100 (in Fig. 6 gezeigt) festgelegtem Schnittführungsblocks 113 durchgeführt, wo führt diese entlang der in Fig. 8 mit DS bezeichneten Resektionslinie des distalen Schnitts, die dann, wie dargestellt aufgrund der Referenzierung auf die Trochlea so liegt, dass durch diesen Schnitt eine durchgehende Fläche zwischen medialer und lateraler Kondyle gebildet ist. Dabei fallen entsprechend Abschnitte an, die von den vorhandenen natürlichen (Rest-)Kondylen stammen. Die so anfallenden Abschnitte werden nun mit einer in Fig. 9 dargestellten einfachen Lehre 140 vermessen, um deren Stärke zu bestimmen, wobei diese Stärke in ein Verhältnis mit der Implantatstärke gesetzt und entsprechend eine Implantatgröße bzw. ein Implantatgrößenparameter abgeleitet werden kann. Hierzu weist die Lehre 140 zwei einander gegenüberliegende Schenkel 142, 143 auf, die zwischen sich eine Keilöffnung 141 einschließen. In diese Keilöffnung kann der Abschnitt AS eingeschoben werden, bis er darin verklemmt. Die Position des Anschlages des Abschnittes AS kann dann auf einer auf dem in der Fig. 9 unten dargestellten Schenkel 143 aufgebrachten Skala 144 abgelesen werden, in diesem Beispiel liegt sie zwischen den Skalawerten 7 und 8. Diese Skalawerte können dabei bereits so gewählt und bemessen sein, dass sie entsprechende Implantatgrößen darstellen und wiedergeben, so dass auf diese Weise eine erste Referenzierung auf die mediale oder laterale Seite erfolgen kann, indem durch Vermessen der beiden Abschnitte AS von der medialen bzw. der lateralen Kondyle festgestellt und bestimmt wird, welche der beiden Kondylen die hierüber ermittelte Implantatgröße bestimmen soll.

Die oben beschriebenen und anhand der Figuren 6 bis 9 aufgezeigten zusätzlichen Instrumente und Vorgehensweisen, die hier als weitere Möglichkeiten zum Vorgehen im gesamten operativen Verfahren, bei dem auch die erfindungsgemäße und vorstehend beschriebene Lehre zum Einsatz kommt, können für sich genommen eigenständige Erfindungen enthalten und insbesondere auch ohne Verbindung mit der hier beschriebenen und beanspruchten erfindungsgemäßen Lehre Verwendung und Gebrauch finden. Sie sind insoweit als eigenständige technische Entwicklungen zu betrachten.

Aus der vorstehenden Beschreibung eines Ausführungsbeispiels der erfindungsgemäßen Lehre sind deren Vorteile und sind die Wirkweise und die Art des Einsatzes dieser Lehre noch einmal deutlich geworden. Selbstverständlich beschränkt die Beschreibung des Ausführungsbeispiels die Erfindung nicht auf dieses spezifische Ausführungsbeispiel. Vielmehr ist die Erfindung in ihrer allgemeinen Tragweite und Bedeutung bestimmt durch die nachfolgenden Ansprüche.

### Bezugszeichenliste

1 Lehre
2 Halte- und Führungsteil
3 Handgriff
4 Gegenstück
5 Führungsschiene
6 Achse
7 Tasterteil
8 Tastarm
9 Tastspitze
10 Anlageteil
11 Anlagestück
12 Anlagestück
13 Fortsatz
14 Fortsatz
15 Querstück
16 Querstück
17 Verbindungspunkt
18 Verbindungspunkt
19 Verbindungspunkt
20 Verbindungspunkt
21 Zeigermarkierung
22 Zeigermarkierung
23 Skalierung
24 Skalierung
25 Zeigermarkierung
26 Zeigermarkierung
27 Durchtrittsöffnung
28 Durchtrittsöffnung
29 Durchtrittsöffnung
30 Klemmöffnung
31 Halte- und Schraubbolzen
32 Hinterschnitt
33 Glattwandiger Abschnitt
34 Achse
35 Verriegelungsstift
36 Ausnehmung
37 Achse
38 Klemmöffnung
39 Seitenwange
40 Winkelskalierung
41 Winkelskalierung
42 Schraubmutter
43 Außengewinde
44 Pinloch
45 Peilöffnung
46 Schwenkstift
47 Schwenklageröffnung
48 Schwenklageröffnung
49 Verriegelungsstift
50 Anschlagfläche
51 Schlittenelement
100 Ausrichtinstrument
101 Anlageplatte
102 Öffnung
103 Stangenaufnahme
104 Intramedullärstange
105 Fortsatz
106 Führungsnut
107 Führungsleiste
108 Schieber
109 Winkelskala
110 Ableseöffnung
111 Oberkante
112 Befestigungsöffnung
113 Schnittführungsblock
114 Doppelstiftriegel
115 Befestigungsstift
116 Öffnung
117 Führungsschlitz
118 Pinloch
119 Gelenkpunkt
120 Anlagestück
130 Ausrichtinstrument
131 Abtastspitze
140 Lehre
141 Keilöffnung
142 Schenkel
143 Schenkel
144 Skala
α Winkelversatz
A Abstand
A₁ Abstand
A₂ Abstand
AP Ausrichtpunkt
AS Abschnitt
DS distaler Resektionsschnitt
DZ Drehzentrum
EK Epikondyle
FFemur
L Linie
OL₁ Orientierungslinie
OL₂ Orientierungslinie
P Planfläche
PK Posteriore Kondyle
RP Referenzpunkt
S Schwenkachse
T Tibia
TR Trochlea

## Patentansprüche

1. Lehre (1) für die Bestimmung einer für einen Patienten passenden Implantatgröße des Femurimplantats einer Knie-Endoprothese, wobei die Lehre (1) folgendes aufweist:
einen Grundkörper (2, 4),
ein gegenüber dem Grundkörper (2, 4) entlang einer Messrichtung (6) verschiebbares Tasterteil (7), wobei das Tasterteil (7) einen Tastarm (8) mit einer Tastspitze (9) für die Anlage an einem anterioren Referenzpunkt (RP) des distalen Femurendes des Patienten aufweist
zwei Anlagestücke (11, 12) für die Anlage an und Referenzierung auf jeweils die mediale und die laterale posteriore Kondyle des distalen Femurendes des Patienten und
wenigstens eine Skalierung (23, 24) und einen zugeordneten Zeiger (21, 22) für die Anzeige einer Lage des Tasterteils (7) in Messrichtung (6) relativ zu wenigstens einem der Anlagestücke (11, 12) für die Angabe einer passenden Implantatgröße,
wobei die Anlagestücke (11, 12) relativ zu dem Grundkörper (2, 4) in einer Weise bewegbar sind, dass ein auf die Messrichtung (6) projizierter Abstand der Tastspitze (9) zu den Anlagestücken (11, 12) für die Anlagestücke (11, 12) derart einstellbar ist, dass dieser Abstand in Bezug auf die beiden Anlagestücke (11, 12) unterschiedlich ist,
und wobei für jedes Anlagestück (11, 12) eine eigene Skalierung (23, 24) und ein zugeordneter Zeiger (21, 22) für die Anzeige einer Lage des Tasterteils (7) in Messrichtung (6) relativ zu dem jeweiligen Anlagestück (11, 12) und für die sich daraus ergebende Angabe einer passenden Implantatgröße vorgesehen sind, **gekennzeichnet durch** eine zwischen den Anlagestücken (11, 12) bestehende Kopplung, die bei einer Relativbewegung des einen Anlagestücks (11, 12) gegenüber dem Grundkörper (2, 4) eine Relativbewegung auch des anderen Anlagestücks (12, 11) gegenüber dem Grundkörper (2, 4) nach sich zieht.

2. Lehre nach Anspruch 1, **gekennzeichnet durch** eine Grundstellung, in der die beiden Anlagestücke (11, 12) einen gleichen auf die Messrichtung (6) projizierten Abstand zu der Tastspitze (9) aufweisen, wobei durch jede aus der Grundstellung herausführende Relativbewegung zwischen einem ersten der Anlagestücke (11, 12) und dem Grundkörper (2, 4) die Kopplung für eine Relativbewegung des zweiten der Anlagestücke (12, 11) und dem Grundkörper (2, 4) sorgt, die zu einer Einstellung der Anlagestücke (11, 12) in der Weise führt, dass diese unterschiedliche auf die Messrichtung (6) projizierte Abstände zu der Tastspitze (9) aufweisen.

3. Lehre nach einem der vorhergehenden Ansprüche, **dadurch gekennzeichnet, dass** die Anlagestücke (11, 12) jeweils einen in der Messrichtung (6) verlaufenden, mit dem Anlagestück (11, 12) fest verbundenen Fortsatz (13, 14) aufweisen, wobei die Fortsätze (13, 14) über zwei zueinander parallel verlaufende Querstücke (15, 16) miteinander verbunden sind, wobei die Querstücke (15, 16) in jeweils einem Verbindungspunkt (17, 18, 19, 20) mit dem jeweiligen Fortsatz (13, 14) gelenkig verbunden sind, so dass die beiden Fortsätze (13, 14) und die beiden Querstücke (15, 16) ein Parallelogramm-Vierpunktgelenk bilden.

4. Lehre nach Anspruch 3, **dadurch gekennzeichnet, dass** die Querstücke (15, 16) deart gelenkig mit dem Grundkörper (2, 4) verbunden sind, dass diese gekoppelt relativ zu diesem verschwenkbar sind.

5. Lehre nach Anspruch 4, **dadurch gekennzeichnet, dass** jedes der Querstücke (15, 16) an einem mittig zwischen den beiden Verbindungspunkten (17, 19; 18, 20), an denen es gelenkig mit jeweils einem der beiden Fortsätze (13, 14) verbunden ist, gelegenen Anbindungspunkt (27, 28) gelenkig mit dem Grundkörper (2, 4) verbunden ist.

6. Lehre nach einem der Ansprüche 4 oder 5, **gekennzeichnet durch** wenigstens eine Winkelskala (40, 41) sowie einen mit dieser zusammenwirkenden Winkelzeiger (25, 26) zum Anzeigen eines Schwenkwinkels, um den die Querstücke (15, 16) relativ zu einer Ausgangsstellung verschwenkt sind.

7. Lehre nach Anspruch 6, soweit dieser mittelbar oder unmittelbar auf Anspruch 2 rückbezogen ist, **dadurch gekennzeichnet, dass** die Ausgangsstellung die Grundstellung ist.

8. Lehre nach einem der vorhergehenden Ansprüche, **gekennzeichnet durch** einen mit dem Grundkörper (2, 4) verbundenen Handgriff (3).

9. Lehre nach einem der vorhergehenden Ansprüche, **gekennzeichnet durch** ein Blockiermittel (42, 43) zum Blockieren von zueinander bewegbaren Teilen der Lehre (1).

## Claims

1. Jig (1) for determining a patient-adapted implant size of the femoral implant of a knee endoprosthesis, wherein the jig (1) comprises the following:
a main body (2, 4),
a probe part (7) displaceable relative to the main body (2, 4) along a measuring direction (6),
wherein the probe part (7) has a probe arm (8) with a probe tip (9) for bearing on an anterior reference point (RP) of the distal end of the patient's femur,
two bearing pieces (11, 12) for bearing on and referencing the medial posterior and the lateral posterior condyle, respectively, of the distal end of the patient's femur, and
at least one scale (23, 24) and an associated indicator (21, 22) for indicating a position of the probe part (7) in the measuring direction (6) relative to at least one of the bearing pieces (11, 12) for specification of a suitable implant size,
wherein the bearing pieces (11, 12) are movable relative to the main body (2, 4) in such a way that a distance of the probe tip (9) to the bearing pieces (11, 12), projected onto the measuring direction (6), is adjustable for the bearing pieces (11, 12) in such a way that this distance is different with respect to the two bearing pieces (11, 12),
and wherein each bearing piece (11, 12) is provided with its own scale (23, 24) and an associated indicator (21, 22) for indicating a position of the probe part (7) in the measuring direction (6) relative to the respective bearing piece (11, 12) and for the resulting specification of a suitable implant size, **characterized by** a coupling between the bearing pieces (11, 12), which coupling, in the case of a relative movement of one bearing piece (11, 12) relative to the main body (2, 4), also causes a relative movement of the other bearing piece (12, 11) relative to the main body (2, 4).

2. Jig according to Claim 1, **characterized by** a basic position in which the two bearing pieces (11, 12) have a same distance to the probe tip (9), projected onto the measuring direction (6), wherein, upon each relative movement between a first of the bearing pieces (11, 12) and the main body (2, 4) leading out of the basic position, the coupling ensures a relative movement of the second of the bearing pieces (12, 11) and the main body (2, 4) leading to an adjustment of the bearing pieces (11, 12) in such a way that they have different distances to the probe tip (9), projected onto the measuring direction (6).

3. Jig according to one of the preceding claims, **characterized in that** the bearing pieces (11, 12) each have an extension (13, 14) which extends in the measuring direction (6) and which is firmly connected to the bearing piece (11, 12), wherein the extensions (13, 14) are connected to each other by means of two crosspieces (15, 16) extending parallel to each other, wherein the crosspieces (15, 16) are connected in an articulated manner to the respective extension (13, 14) at a respective connection point (17, 18, 19, 20), such that the two extensions (13, 14) and the two crosspieces (15, 16) form a parallelogram four-point joint.

4. Jig according to Claim 3, **characterized in that** the crosspieces (15, 16) are connected in an articulated manner to the main body (2, 4) in such a way that the crosspieces (15, 16), in a coupled state, are pivotable relative to the main body (2, 4) .

5. Jig according to Claim 4, **characterized in that** each of the crosspieces (15, 16) is connected in an articulated manner to the main body (2, 4) at an attachment point (27, 28) situated centrally between the two connection points (17, 19; 18, 20) at which it is connected in an articulated manner to one of the two extensions (13, 14), respectively.

6. Jig according to either of Claims 4 and 5, **characterized by** at least one angle scale (40, 41) and by an angle indicator (25, 26) which cooperates with the latter for indicating a pivot angle about which the crosspieces (15, 16) are pivoted relative to a starting position.

7. Jig according to Claim 6, in so far as the latter refers back directly or indirectly to Claim 2, **characterized in that** the starting position is the basic position.

8. Jig according to one of the preceding claims, **characterized by** a handle (3) connected to the main body (2, 4).

9. Jig according to one of the preceding claims, **characterized by** a blocking means (42, 43) for blocking parts of the jig (1) that are movable relative to each other.

## Revendications

1. Jauge (1) pour déterminer la taille, adaptée à un patient, d'un implant fémoral d'une endoprothèse du genou, la jauge (1) présentant :
un corps de base (2, 4),
une partie de palpeur (7) pouvant être déplacée par rapport au corps de base (2, 4) le long d'une direction de mesure (6), la partie de palpeur (7) présentant un bras de palpage (8) avec une pointe de palpage (9) destinée à s'appliquer contre un point de référence antérieur (RP) de l'extrémité distale du fémur du patient,
deux pièces d'appui (11, 12) pour l'application à chaque fois contre, et le référencement les à chaque fois aux condyles postérieurs médial et latéral de l'extrémité distale du fémur du patient et
au moins une échelle (23, 24) et une aiguille associée (21, 22) pour afficher une position de la partie de palpeur (7) dans la direction de mesure (6) par rapport à au moins l'une des pièces d'appui (11, 12) pour l'indication d'une taille d'implant adaptée,
les pièces d'appui (11, 12) pouvant être déplacées par rapport au corps de base (2, 4) de telle sorte qu'une distance projetée sur la direction de mesure (6) de la pointe de palpage (9) aux pièces d'appui (11, 12) puisse être ajustée pour les pièces d'appui (11, 12) de telle sorte que cette distance soit différente par rapport aux deux pièces d'appui (11, 12),
et une échelle propre (23, 24) et une aiguille associée (21, 22) pour l'affichage une position de la partie de palpeur (7) dans la direction de mesure (6) par rapport à la pièce d'appui respective (11, 12) et pour l'indication résultante d'une taille d'implant adaptée étant prévues pour chaque pièce d'appui (11, 12), **caractérisée par** un accouplement existant entre les pièces d'appui (11, 12), qui, dans le cas d'un mouvement relatif de l'une des pièces d'appui (11, 12) par rapport au corps de base (2, 4), entraîne également un mouvement relatif de l'autre pièce d'appui (12, 11) par rapport au corps de base (2, 4) .

2. Jauge selon la revendication 1, **caractérisée par** une position de base dans laquelle les deux pièces d'appui (11, 12) présentent une distance identique à la pointe de palpage (9), projetée sur la direction de mesure (6), l'accouplement assurant un mouvement relatif de la deuxième des pièces d'appui (12, 11) et du corps de base (2, 4) par chaque mouvement relatif hors de la position de base entre une première des pièces d'appui (11, 12) et le corps de base (2, 4), ledit mouvement relatif conduisant à un ajustement des pièces d'appui (11, 12) de telle sorte que celles-ci présentent des distances différentes à la pointe de palpage (9), en projection sur la direction de mesure (6).

3. Jauge selon l'une quelconque des revendications précédentes, **caractérisée en ce que** les pièces d'appui (11, 12) présentent à chaque fois une saillie (13, 14) connectée fixement à la pièce d'appui (11, 12), s'étendant dans la direction de mesure (6), les saillies (13, 14) étant connectées l'une à l'autre par le biais de deux pièces transversales (15, 16) s'étendant parallèlement l'une à l'autre, les pièces transversales (15, 16) étant connectées de manière articulée dans un point de liaison respectif (17, 18, 19, 20) à la saillie respective (13, 14) de telle sorte que les deux saillies (13, 14) et les deux pièces transversales (15, 16) forment un parallélogramme articulé à quatre points.

4. Jauge selon la revendication 3, **caractérisée en ce que** les pièces transversales (15, 16) sont connectées de manière articulée au corps de base (2, 4) de telle sorte que celles-ci puissent pivoter de manière accouplée par rapport à celui-ci.

5. Jauge selon la revendication 4, **caractérisée en ce que** chacune des pièces transversales (15, 16) est connectée de manière articulée au corps de base (2, 4) au niveau d'un point de raccordement (27, 28) situé centralement entre les deux points de liaison (17, 19 ; 18, 20) au niveau desquels elle est connectée de manière articulée à chaque fois à l'une des deux saillies (13, 14).

6. Jauge selon l'une quelconque des revendications 4 ou 5, **caractérisée par** au moins une échelle angulaire (40, 41) ainsi que par un indicateur d'angle (25, 26) coopérant avec celle-ci pour indiquer un angle de pivotement autour duquel les pièces transversales (15, 16) sont pivotées par rapport à une position de départ.

7. Jauge selon la revendication 6, dans la mesure où celle-ci se rapporte directement ou indirectement à la revendication 2, **caractérisée en ce que** la position de départ est la position de base.

8. Jauge selon l'une quelconque des revendications précédentes, **caractérisée par** une poignée (3) connectée au corps de base (2, 4).

9. Jauge selon l'une quelconque des revendications précédentes, **caractérisée par** un moyen de blocage (42, 43) pour bloquer des parties de la jauge (1) pouvant être déplacées les unes par rapport aux autres.
